# EUROPEAN PATENT SPECIFICATION

(11) **EP 4 076 315 B1**
(45) Date of publication and mention of the grant of the patent: **14.05.2025**
(21) Application number: 19828661.9
(22) Date of filing: 16.12.2019
(51) Int. Cl.: A61F 13/15, A61F 13/56, A61L 15/62

(54) **A HYGIENIC ABSORBENT ARTICLE COMPRISING A FASTENING MEMBER**
ABSORBIERENDER HYGIENEARTIKEL MIT EINEM BEFESTIGUNGSELEMENT
ARTICLE ABSORBANT HYGIÉNIQUE COMPRENANT UN ÉLÉMENT DE FIXATION

(43) Date of publication of application: 26.10.2022
(73) Proprietor: Essity Hygiene and Health Aktiebolag, 405 03 Göteborg (SE)
(72) Inventor: SILFVERSTRAND, Anders, 405 03 Göteborg (SE); KLING, Robert, 405 03 Göteborg (SE)
(74) Representative: Hoffmann Eitle
(86) International application number: PCT/EP2019/085390
(87) International publication number: WO 2021/121544

(56) References cited:
- EP-A2- 0 809 952
- EP-A2- 0 893 114
- WO-A1-2009/060893
- WO-A1-2010/081042
- JP-A- 2006 325 939
- JP-A- 2009 172 599
- JP-A- H11 181 306
- JP-B2- 3 389 078

## Description

### TECHNICAL FIELD

The present disclosure pertains to a fastening member for a hygienic absorbent article. The fastening member comprises a water-soluble resin. The disclosure also relates to a hygienic absorbent article comprising the fastening member, to a method for treating the hygienic absorbent article comprising the fastening member and to use of the fastening member for the manufacture of a hygienic absorbent article and/or a disposal bag.

### BACKGROUND

Hygienic absorbent articles such as diapers are usually folded or rolled-up after use so that the soiled portion is wrapped inside the folded or rolled-up article, making it suitable for disposal. In order to prevent the soiled article from unfolding and to keep the soiled portion inside, it is desired that fastening means are arranged to keep the article in the folded or rolled-up state for disposal.

EP 0 893 114 discloses a disposable diaper with disposal securing means. It is disclosed that male and/or female engaging elements of a first and/or second surface fastener member are made of a water-soluble resin. The water-soluble resin may be polyvinyl alcohol, decomposed polyvinyl alcohol, polyacrylic acid, polyethylene oxide, CMC and gum. When water or alcohol solution is applied or sprayed over the first and/or second surface fastener member the water-soluble resin dissolves and becomes adhesive, allowing the fastener member to be used for securing a used and rolled-up diaper in the rolled configuration such that the rolled-up diaper can be safely handled as waste.

In addition to convenient handling of used hygienic articles, increasing environmental concerns have created a demand for hygienic absorbent articles such as diapers that also are more environmentally friendly.

Conventionally, diapers are treated as waste and sent to waste handling stations where they are disposed of by e.g. land filling or incineration. Unfortunately, this is associated with problems such as long degradation time and emission of greenhouse gases such as methane and carbon dioxide. In order to mitigate these problems hygienic articles comprising biodegradable materials have been developed.

WO 2009/060893 discloses a disposable diaper which comprises a biodegradable and water-soluble material. It is disclosed that the diaper comprises an outer sheet, an inner nonwoven fabric sheet, a pulp material and an adhesive which are all formed of a water-soluble material which is biodegradable and melts at a temperature which is higher than the human body temperature, for example polyvinyl alcohol. It is also disclosed that the diaper may comprise an engaging member formed of polypropylene nonwoven fabric.

EP 0893114 A2 discloses a disposable diaper that has a pair of first surface fastener members supported by a rear flap of a diaper body and a second surface fastener member supported by a front flap of the diaper body. The first and second surface fastener members have first and second engaging elements; the first and/or second engaging elements are made of water-soluble resin. After approximately 1/3 to 2/5 of the diaper body on the side of the rear flap is folded inwardly, the diaper body is wound into a roll toward the front flap, and then the water-soluble resin of the first and/or second engaging elements is dissolved with water. Then the first surface fastener members are pressed against the second surface fastener member to fasten the diaper body in roll as waste.

Further progress has been made to make the use and disposal of hygienic absorbent articles such as diapers even more environmentally friendly by the creation of central recycling centers for such articles. The recycling process involves collection of the absorbent articles from people's homes, hospitals, retirement homes, pre-schools, city recycling centres or other suitable locations followed by transport to the recycling center where treatment of the collected articles takes place. Depending on the processes being used, part or all of the component materials in the absorbent articles may be recovered. Examples of raw materials that may be recovered include cellulose, plastics and superabsorbent polymers. The raw materials may subsequently be recycled into an appropriate material or article thereby reducing the negative impact on the environment.

Additionally or alternatively, the hygienic absorbent articles may be treated in specialized on-site washing machines in e.g. people's homes or institutions such as day care centres or hospitals. Subsequently, the treated articles may be separated into different materials followed by recycling of said materials.

The shift towards a more sustainable and environmentally friendly handling of used hygienic articles such as diapers has created a need for such articles which are recoverable and/or recyclable in a convenient way. Further, it is desired that the user or caregiver may easily package the article after use into a configuration that is stable until subjected to treatment for recovery and/or recycling.

### SUMMARY

It is an object of the present disclosure to provide a hygienic article comprising a fastening member which fastening member resists loss of functionality during ordinary use and yet allows for being disposed, recovered and/or recycled in a treatment process taking place after use.

One or more of the above objects may be achieved with a hygienic article as defined in the claims. Further embodiments are set out in the dependent claims, in the following description and in the drawings.

Thus, the present disclosure provides a hygienic absorbent article comprising a fastening member which comprises a water-soluble resin, wherein the water-soluble resin dissolves in an aqueous solution having a temperature equal to or above 40, about 60 or about 80 degrees Celsius, wherein the fastening member is disintegrated and/or dissolved at a temperature equal to or above 40, about 60 or about 80 degrees Celsius while remaining undissolved at a temperature below 40 degrees Celsius. For instance, the aqueous solution has a temperature from 40 degrees Celsius to about 100 degrees Celsius. As used herein, an aqueous solution is a solution comprising water and optionally a further solvent. The further solvent may be a water-miscible solvent. For instance, the water-miscible solvent may be an alcohol such as ethanol.

The water-soluble resin of the fastening member allows it to be dissolved such as fully dissolved or at least dissolved to a degree where the fastening properties of the fastening member are lost when contacted with an aqueous solution having a temperature equal to or above 40, 60 or 80 degrees Celsius, i.e. from 40 degrees Celsius to 100 degrees Celsius, such as from about 60 degrees Celsius to 100 degrees Celsius, such as from about 80 degrees Celsius to 100 degrees Celsius, while remaining undissolved or substantially undissolved a temperature below 40, 60 or 80 degrees Celsius. This regulation of the water solubility of the fastening member allows the fastening member to resist loss of fastening functionality, such as loss of fastening functionality by e.g. dissolution, at a temperature below 40, about 60 or about 80 degrees Celsius. For instance, the fastening member as disclosed herein resists deterioration at room temperature caused by contact with an aqueous solution like urine or inadvertent wetting of the fastening member as may occur when manipulating the fasteners with wet hands when changing a soiled article for a new fresh one. Hence, this is a significant benefit since contact with an aqueous solution frequently takes place. For instance, this may happen accidentally upon e.g. urine leakage from a diaper. In a further example, some hygienic absorbent articles such as swim diapers are intended to be used together with water and their components should then resist dissolution.

The water-soluble resin of the fastening member may be selected from one or more of the following polymers: polyvinyl alcohol, polyacrylic acid, polyethylene oxide, carboxymethyl cellulose, or a copolymer of any one of the foregoing polymers, or a combination of any one of the foregoing polymers or copolymers. The water-soluble resin comprises polyvinyl alcohol and may consist of polyvinyl alcohol. The choice of polyvinyl alcohol is advantageous, since it is biodegradable, readily available, and exists in different grades allowing for selection of a grade associated with the properties that are desired.

Generally, polyvinyl alcohol is a water-soluble synthetic polymer of formula [CH₂CH(OH)]ₙ, wherein n is the number of monomers of the polymer, and it is biodegradable under both aerobic and anaerobic conditions. Commonly, polyvinyl alcohol is prepared by hydrolysis of polyvinyl acetate to a desired degree which impacts the water solubility of the resulting polyvinyl alcohol. For instance, only polyvinyl alcohol with a low degree of hydrolysis dissolves in cold water or water having room temperature while polyvinyl alcohol with a high degree of hydrolysis only dissolves in hot water. Further, depending on the application the polyvinyl alcohol may be prepared to have a desired molecular weight, to be in an amorphous, semicrystalline or crystalline state, to exhibit a particular melting point or glass transition temperature etc.

Thus, the polyvinyl alcohol of the fastening member disclosed herein is derived from polyvinyl acetate such as hydrolyzed polyvinyl acetate. Further, the polyvinyl alcohol described herein has a degree of hydrolysis from 70 mole % to 100 mole %, such as from 85 mole% to 100 mole%, such as from 70 mole% to 90 mole%, such as from 70 mole% to 80 mole%. In this range, the polyvinyl alcohol does not dissolve when contacted with water or an aqueous solution having a temperature below 40 degrees Celsius such as room temperature. For instance, the polyvinyl alcohol may have a degree of hydrolysis from 99 mole% to 100 mole %, which may be considered to be super hydrolyzed polyvinyl alcohol. In a further example, the polyvinyl alcohol may have a degree of hydrolysis from 98 mole% to 99 mole %, which may be considered to be fully hydrolyzed polyvinyl alcohol. In still a further example, the polyvinyl alcohol may have a degree of hydrolysis from 90 mole% to 98 mole %, which may be considered to be intermediately hydrolyzed polyvinyl alcohol. In still a further example, the polyvinyl alcohol may have a degree of hydrolysis from 85 mole% to 90 mole %, which may be considered to be partially hydrolyzed polyvinyl alcohol. By selecting a polyvinyl alcohol with a degree of hydrolysis in the range of from 85 mole % to 100 mole % a fastening member comprising the polyvinyl alcohol maintains its integrity and fastening functionality and resists or substantially resists dissolution when contacted with an aqueous solution having a temperature below about 65 degrees Celsius such as room temperature while allowing it to be dissolved when contacted with water or an aqueous solution having a temperature above about 65 degrees Celsius. In this way, a fastening member and/or a hygienic article comprising the fastening member may conveniently be used under normal user conditions and still be readily recovered and/or recycled in a subsequent treatment process.

Moreover, by selecting polyvinyl alcohol having a high degree of hydrolysis, such as super hydrolyzed or fully hydrolyzed polyvinyl alcohol, other desirable properties may also be obtained such as no or little foaming upon contact with water. As a result, inclusion of an antifoaming agent into the fastening member may be avoided. Of course, this simplifies manufacture, lowers costs and reduces the overall environmental impact. Thus, the present disclosure provides a fastening member as described herein which does not comprise an antifoaming agent.

The polyvinyl alcohol of the fastening member disclosed herein may be amorphous, semicrystalline, crystalline or a mixture thereof. In an example, the polyvinyl alcohol exhibits a glass transition temperature (T_{g}) in the range of from 58 degrees Celsius to 85 degrees Celsius. Further, the polyvinyl alcohol may have molecular weight in the range of from 13.000 g/mole to 186.000 g/mole. Additionally or alternatively, the molecular weight may be expressed as an aqueous solution viscosity such as a 4 wt% aqueous solution viscosity ranging from about 3 cps to about 72 cps. Depending on the desired properties, polyvinyl alcohol with desired characteristics may be selected. For example, high viscosity is often associated with increased adhesive strength and/or less water sensitivity.

The fastening member described herein may be a disposal fastening member for a hygienic absorbent article such as a diaper. A disposal fastening member is used for securing a hygienic absorbent article in a folded or rolled-up disposal configuration. The disposal fastening member allows for preventing a soiled absorbent article to be unfolded after use so that the soiled portion remains inside the hygienic article until it is subjected to a treatment process such as a treatment process described herein.

The fastening member as disclosed herein may be a fastening member having a primary function as a fastener for securing a hygienic absorbent article in a pant-like configuration on a user. Such fastening member may have a secondary function as a disposal fastening member, optionally in combination with one or more dedicated disposal fastening members.

The present disclosure also provides a hygienic absorbent article such as a diaper comprising the fastening member described herein. The diaper may be an open type diaper. Additionally or alternatively, the hygienic absorbent article may be selected from the group consisting of a pant type diaper, a sanitary napkin, an incontinence guard, a seat protector or a bed protector.

A fastening member as disclosed herein may be a fastening tab on which fastener elements are applied. The fastening member may comprise or consist of a water-soluble resin, wherein the fastening member disintegrates or dissolves in an aqueous solution having a temperature equal to or above 65 degrees Celsius, the fastening member disintegrating or dissolving to such extent that the fastening member loses its fastening ability. In the fastening member as disclosed herein, it may be sufficient if a component of the fastening member disintegrates or dissolves in order to render the fastening member non-functional. The fastening tabs are usually made from a material different from that of the fastener elements and may serve as a backing or substrate to which the fastener elements are attached with a construction bond. The construction bond is not intended to be broken during use of the hygienic article.

In a fastening member as disclosed herein, fastening elements which are part of the fastening member, e.g. hook elements, fastening adhesive, loop elements, may comprise or consist of the water-soluble resin. Furthermore, a substrate included in the fastening member may comprise or consist of the water-soluble resin. A further option is that the construction bond between a fastening tab and fastening elements is dissolvable. Any combination of such dissolvable components are also conceivable in a fastening member as disclosed herein. In all instances, the technical effect for a fastening member which is being used as a disposal fastening member for securing a hygienic article in a disposal configuration, is that the fastening ability for the fastening member is lost when the article is exposed to a hot water solution. Thus, the fastening member is detached and the article may be unfolded from the disposal configuration and subjected to recycling treatment.

The fastening members as disclosed herein may be mechanical fastening members with cooperating fastening elements. A common type of mechanical fastening members are fastening members which are referred to as "male-type fastening members" or "hook-type fastening members". Mechanical fastening members which are used for fastening together a hygienic absorbent article such as a diaper into a pant-like configuration are commonly of the type with a first fastening member having male fastening elements, such as hooks or prongs which cooperate with a female fastening element such as a loop-type fastening element to create an openable and reclosable fastening between the first and second fastening member. However, hook-type fastening members which are arranged to be fastened together with another hook-type fastening member of the same or a different configuration are also known in the art.

Loop-type female fastening elements may be provided in the form of a nonwoven web, where fibres or strands in the nonwoven web act as loops in which the corresponding male fastening elements may be caught.

Male or hook-type fastening elements are usually molded plastic structures, with a plurality of small protrusions provided with loop-engaging heads. Such structures are generally referred to in the art as hook-type fastening elements even though the loop-engaging heads may take other forms such as a mushroom-shape or the like.

The hook-type fastening elements are comparatively more expensive than the loop-type materials and are commonly applied to a substrate, such as a fastening tab in the form of a patch of hook-type fastening material.

A hook-type fastening member may be arranged to be attached to a surface of the hygienic absorbent article which is composed of a suitable nonwoven material which may provide loop-type fastening elements. Such material may form all or part of an outer cover sheet of the hygienic absorbent article or may be applied as a dedicated "landing zone" in an area of the hygienic absorbent article where the hook-type fastening member is intended to be fastened.

A fastening member as disclosed herein may be an adhesive fastening member. An adhesive fastener is commonly arranged to cooperate with a dedicated landing zone on the hygienic absorbent article, the landing zone being made from a material which can withstand repeated fastening and unfastening of the adhesive fastening member without being damaged.

The fastening member as disclosed herein may have any kind of fastening elements as known in the art disposed therein, such as a male-type mechanical fastening elements, female-type mechanical fastening elements or adhesive fastening elements.

The fastening member may take the form of a fastening tab formed of a substrate material having fastening elements disposed thereon, the substrate material comprising or consisting of a water-soluble resin, such that the substrate material disintegrates and/or dissolves in an aqueous solution having a temperature equal to or above 40 degrees Celsius.

The hygienic absorbent article described herein comprises an absorbent core 1 disposed between an inner coversheet 2 and an outer coversheet 3. The fastening member described herein may be adapted to fasten to at least a portion of the outer coversheet 3 which allows the article to be secured in a compact disposal configuration as described herein. The hygienic article may comprise a carrier material to which the fastening member is attached. For example, the carrier material may comprise or consist of a water-soluble resin such as a water-soluble resin described herein. The outer cover sheet of the hygienic article described herein may comprise a landing zone or may comprise or consist of a material adapted to attach the fastening member.

The outer cover sheet and/or a landing zone on the outer cover sheet may comprise or consist of a nonwoven material having fibre loops which may serve to engage male fastening elements on the fastening member as disclosed herein for forming an attachment between the fastening member and the outer cover sheet of the hygienic article. Such attachment is referred to herein as a "hook-and-loop" attachment and is generally a releasable attachment implying that the fastening member can be detached from the outer cover sheet or landing zone without functional loss to the fastening member or the outer cover sheet or landing zone. It is generally preferred that a fastening member, such as a hook-type fastening member can be repeatedly attached and detached such that the fastening member may be repositioned on the outer coversheet or landing zone as desired.

Additionally or alternatively, the outer coversheet may comprise a disposal fastening member as described herein.

The hygienic absorbent article described herein may further comprise a water-soluble disposal bag. The water-soluble disposal bag may comprise or consist of a water-soluble resin as described herein and/or comprise a fastening member as described herein. Such a water-soluble disposal bag may be used to enclose the hygienic absorbent article prior to and/or after use thereof. This may be convenient for a user or a caregiver who wants to be able to easily pack it into a bag or pocket to have it easily accessible when desired such as when being away from home. Further, a used hygienic article may be enclosed in the water-soluble disposal bag to provide extra protection against odour, contact with the e.g the interior of a bag or pocket where it is temporarily stored prior to being disposed of and/or provide a convenient packaging for treatment in a subsequent treatment process such as a treatment process described herein. Conveniently, the absorbent article may be provided in the form of a kit comprising (i) said absorbent article, (ii) said water-soluble disposal bag and (iii) optionally instructions for use. For example, the instructions for use may explain how to enclose the used hygienic article in the disposal bag and/or how to fasten the fastening member.

The disposal bag described herein may have a size making it suitable for accommodating one or more hygienic absorbent articles such as a diaper described herein. For instance, the disposal bag may have a size making it suitable for accomodating from 1 to 10 hygienic absorbent articles. In an example, the disposal bag may have a size making it suitable for accomoding a single hygienic absorbent article.

Additionally or alternatively, there is also described a disposal bag for a hygienic absorbent article such as a baby diaper. The disposal bag resists loss of functionality during ordinary use and yet allows for being disposed, recovered and/or recycled in a treatment process taking place after use.

The present disclosure also provides a method for treating a hygienic absorbent article such as an absorbent article described herein, i.e. a treatment method. The method comprises the steps of:
a) subjecting a hygienic absorbent article as described herein to an aqueous solution as described herein at a temperature equal to or above about 40, about 60 or about 80 degrees Celsius,
b) allowing the fastening member and optionally any further components of the absorbent article that are soluble in the aqueous solution to disintegrate and/or dissolve in the aqueous solution thereby providing (i) a further aqueous solution and (ii) absorbent article components not soluble in the aqueous solution, and
c) separating (i) and (ii) of step b) from each other.

The hygienic absorbent article of step a) may be a used hygienic absorbent article which is secured in a compact configuration for disposal as described herein. For instance, the used hygienic article may be in a folded or rolled up configuration in which the soiled portion is wrapped inside and the configuration is secured by means of the fastening member. The compact configuration may be provided by a consumer or a caregiver and allows for a convenient handling as well as a providing the article in a form suitable for the treatment process described herein. As explained herein, the water-soluble resin of the fastening member does not dissolve or substantially not dissolve when contacted with an aqueous solution at a temperature below 40, about 60 or about 80 degrees Celsius such as room temperature. As a result, its function remains unchanged or substantially unchanged so that the compact configuration is maintained until the fastening member is contacted with an aqueous solution having a temperature equal to or above 40 degrees Celsius, i.e. from 40 degrees Celsius to 100 degrees Celsius, such as from about 60 degrees Celsius to 100 degrees Celsius, such as from about 80 degrees Celsius to 100 degrees Celsius. When this temperature is reached the fastening member and optionally any further components of the absorbent article that are soluble in the aqueous solution disintegrate and/or dissolve in the aqueous solution thereby providing (i) a further aqueous solution and (ii) absorbent article components not soluble in the aqueous solution, i.e. step b) of the method described herein. It will be appreciated that the dissolution in the aqueous solution may be expedited by e.g. performing stirring such as mechanical stirring in any of the method steps. Thus, the method described herein may comprise stirring and dissolving the fastening member and optionally any further components of the hygienic absorbent article that are soluble in the aqueous solution. The stirring and dissolution may take place in a processor such as a processor having a rotation drum.

A further effect associated with the water-soluble resin of the fastening member described herein is that it does not or substantially does not produce foam upon contact with an aqueous solution. This is an advantage, since foaming makes efficient processing more difficult. As a result, no foaming agent may have to be added to the method. Thus, there is provided a method as described herein which does not comprise an antifoaming agent.

Thus, the further aqueous solution comprising the dissolved fastening member and any further dissolved components of the hygienic article may be separated from absorbent article components that are not soluble in the aqueous solution, i.e. step c) of the method described herein. For instance, step c) may involve filtration, centrifugation, flotation and any combination thereof. The separated aqueous solution may be subjected to solvent removal by e.g. distillation and/or evaporation.

Additionally, the method described herein may comprise a step d):
d) recovering, recycling and/or disposing (i) and/or (ii). This method step allows for reuse of at least part of the components and/or materials of the hygienic absorbent articles subjected to the treatment process. Thus, the method described herein may be a method for recovery and/or recycling of a hygienic absorbent article. For example, plastic parts and/or superabsorbent material may be recovered and/or recycled using the method described herein. As a result, the waste and negative environmental impact associated with the use of hygienic absorbent articles is reduced.

The method described herein may further comprise one or more of sterilizing, shredding, drying. For example, the hygienic absorbent articles and/or any components or materials produced in the method may be subjected to sterilization, shredding and/or drying.

### DEFINITIONS

As used herein, the term "cps" stands for centipoise.

The term "room temperature" stands for a temperature from about 20 degrees Celsius to about 25 degrees Celsius.

The terms "recycled", "recyclable" etc. refer to a process of converting materials, components or products into new materials, components and/or objects for a purpose which may be the same or different as the original purpose.

The term "recover", "recoverable" etc. refers to collecting or extracting materials, components or products for reuse such as reuse for a purpose which may be the same or different as the original purpose.

The term "disposal" generally refers to getting rid of an object, component and/or material. For instance, the disposal may include incineration and/or landfilling.

The expression "aqueous solution" refers to a solution comprising water and optionally a solvent such as a water miscible solvent. The water miscible solvent may be an alcohol such as ethanol.

The term "water-soluble" as used herein refers to dissolution of a component in water or an aqueous solution when examined by e.g. ocular inspection, i.e. inspection by the naked eye. The ocular inspection may take place at a desired temperature such as room temperature or at a temperature from about 65 degrees Celsius to about 100 degrees Celsius. The water solubility may be determined as described herein.

The expression "compact disposal configuration" as used herein refers to a configuration of an article such as a hygienic absorbent article ... that is stable and allows for a convenient handling until subjected to treatment for recovery and/or recycling.

### BRIEF DESCRIPTION OF THE DRAWINGS

The present disclosure will be further explained hereinafter by means of non-limiting examples and with reference to the appended drawings wherein:
- Figure 1: shows an open type diaper provided with fastening members as described herein.
- Figure 2: shows an open type diaper according to Figure 1 rolled up into a compact configuration;
- Figures 3A - 3E: show the procedure in which the diaper of Figure 1 is folded into a compact configuration;
- Figure 4: shows a pant type diaper provided with a disposal fastening member as described herein;
- Figure 5: shows the pant type diaper 1 of Figure 4 which has been brought into a rolled up configuration wherein the disposal fastening member 5 attaches to the outer coversheet 4; and
- Figures 6A to 6C: show the diaper of Figure 3D before and after it has been subjected to an aqueous solution having a temperature equal to or above 40 degrees Celsius.
- Figure 7A: shows a disposal bag in an open configuration.
- Figure 7B: shows a side view of a disposal bag 101 when it is in a configuration about to be closed.

### DETAILED DESCRIPTION

It is to be understood that the drawings are schematic and that individual components, such as layers of material are not necessarily drawn to scale. The hygienic absorbent articles shown in the figures are provided as examples only and should not be considered limiting to the invention as disclosed herein. As set out herein, the fastener member as disclosed herein is applicable to any type of hygienic absorbent article which is folded or rolled-up and fastened in a disposal configuration after use, such as pant-type absorbent articles of the open or closed kind, sanitary napkins, incontinence protectors, bed protectors, seat protectors, etc.

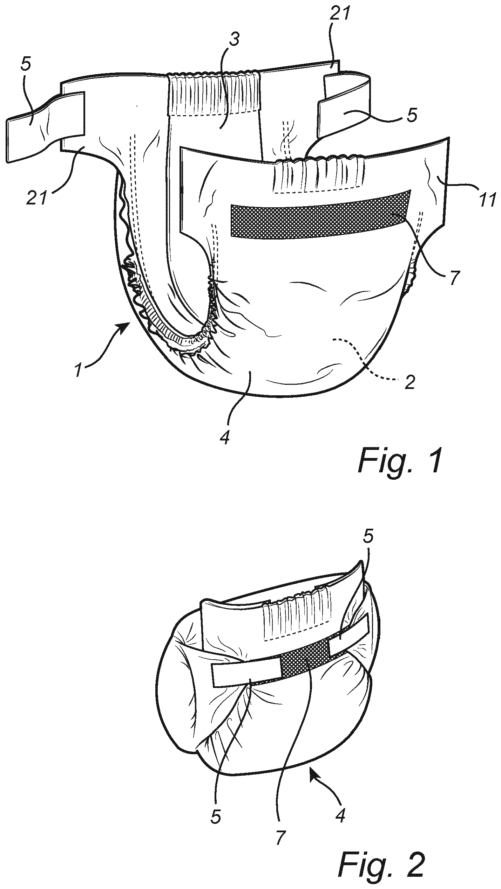

A hygienic absorbent article provided with a fastening member in accordance with the present disclosure is shown in Figure 1. The diaper 1 comprises an absorbent core 2 that is disposed between an inner coversheet 3 and an outer coversheet 4. Further, the diaper 1 comprises first fastening members 5, and a second fastening member 7 located on the outer coversheet 4. The first fastening members 5 and/or the second fastening member 7 may comprise a water-soluble resin as described herein, Thus, the first fastening members 5 and/or the second fastening member 7 may be a fastening member in accordance with the present disclosure. It will be appreciated that the second fastening member 7 may be replaced with a landing zone as described herein.

Figure 2 shows the hygienic absorbent article of Figure 1 that is rolled up into a compact disposal configuration. It will be appreciated that this configuration makes the hygienic absorbent article easy and hygienic to handle and transport and suitable for being collected and subjected to a treatment process as described herein.

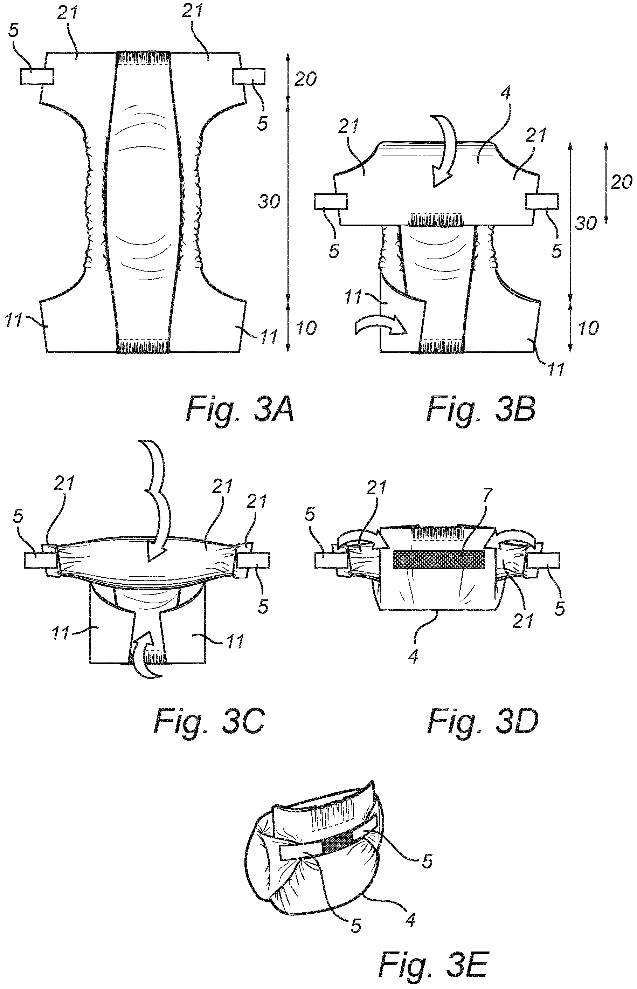

Figures 3A - 3E show the procedure in which the diaper of Figure 1 is folded into a compact configuration. Figure 3A is a top inner view of the diaper 1. The diaper 1 has a rear portion 20, a crotch portion 30, a front portion 10 and first fastening members 5 which are positioned on the rear portion 20 of the diaper 1. It is to be understood that the fastening members 5 could equally well be positioned on the front portion 11 of the diaper 1. Figure 3B shows the diaper of Figure 3A with the rear portion 20 folded inwardly so as to cover the entire area of the crotch portion 30. At the same time side flaps 11 of the front portion 10 are folded inwardly onto the inner cover sheet 3. Then the diaper 1 is wound with the folded edge of the rear portion 20 acting as a core, as a roll toward the front portion 10 as shown in Figure 3C. This winding or rolling-up is continued until the second fastening member 7 which is arranged on the outer surface of the front portion 10 comes to the outside of the roll as shown in Figure 3D. At that time, side flaps 21 of the rear flap 20 and the two first fastening members 5 extend laterally in opposite directions from opposite ends of the roll of the diaper 1 as shown in Figure 3D. Finally, the first fastening members 5 are folded inwardly over the rolled diaper 1 and are attached to the second fastening member 7 which is exposed on the outside of the roll, as shown in Figure 3E. It will be appreciated that the second fastening member 7 may be replaced with a landing zone as described herein.

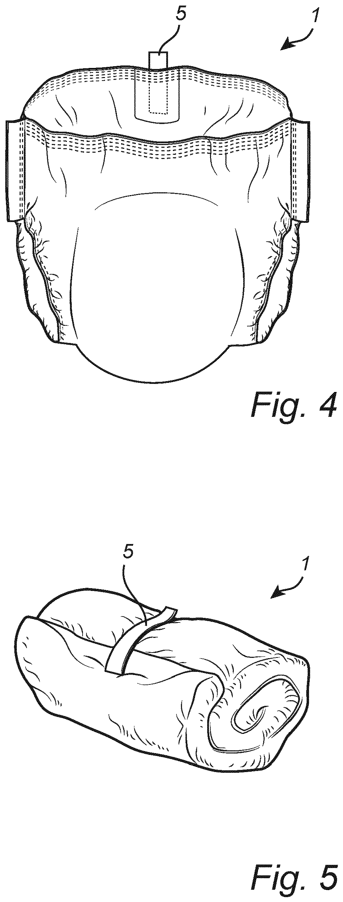

Figure 4 shows a pant type diaper 1 provided with a disposal fastening member 5 as described herein. The fastening member 5 is located on an outer coversheet 4 of the diaper 1.

Figure 5 shows the pant type diaper 1 of Figure 5 which has been brought into a rolled up configuration wherein the disposal fastening member 5 attaches to the outer coversheet 4.

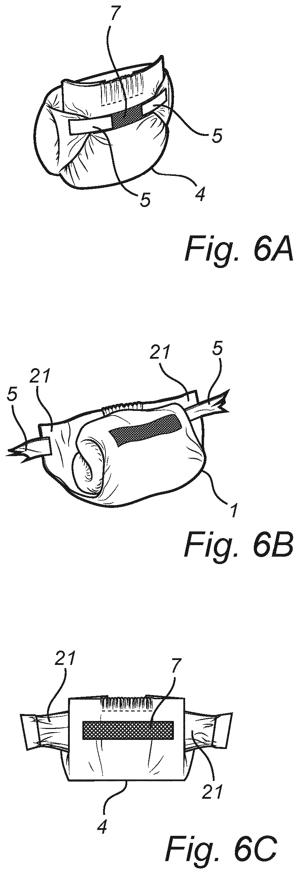

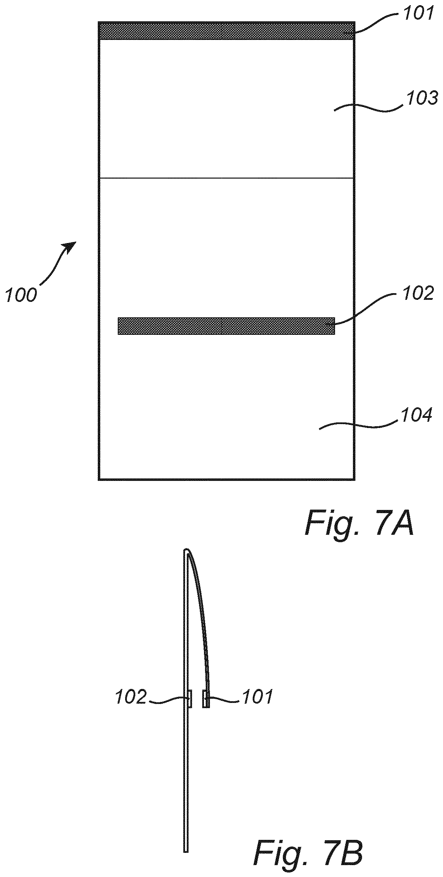

Figures 6A to 6C show the diaper 1 of Figure 3E before and after it has been subjected to an aqueous solution having a temperature equal to or above 40 degrees Celsius, such as from 65 degrees Celsius to 100 degrees Celsius. Figure 6A shows the diaper 1 in Figure 3E before exposure to the aqueous solution. Figure 6B shows the diaper 1 after exposure to the aqueous solution, wherein the first fastening members 5 have been partly dissolved and are detached from the second fastening member 7 thereby opening the diaper 1. The configuration which is assumed by the diaper 1 in Figure 6C follows on the configuration assumed by the diaper 1 in Figure 7B and shows that the first fastening members 5 eventually are dissolved in the aqueous solution. Thus, figures 6A-6C show the initial steps of the treatment method described herein and may be followed by further method steps as described herein.

Figure 7A shows a disposal bag 101 in an open configuration. The disposal bag 101 comprises a closure flap 103 and a pouch portion 104. The closure flap 103 is provided with a fastening member 101. The pouch portion 104 is provided with a fastening member 102. The fastening members 101 and 102 are configured to be able to attach to each other when the closure flap 103 is folded over the pouch portion 104. For instance, the fastening member 101 may comprise hooks and the fastening member 102 may comprise loops or vice versa. Alternatively or additionally, the fastening member 101 and/or the fastening member 102 may comprise an adhesive. The hooks, loops and/or adhesive may be arranged on a carrier material. Further, the fastening member 101 and/or the fastening member 104 may comprise or consist of a water-soluble resin as described herein. Thus, the fastening member 101 and/or the fastening member 104 may comprise or consist of a water-soluble resin, wherein the water-soluble resin dissolves in an aqueous solution having a temperature equal to or above 40 degrees Celsius, wherein the fastening member is disintegrated and/or dissolved at a temperature equal to or above 40 degrees Celsius. Additionally or alternatively, the disposal bag may comprise or consist of a water-soluble resin as described herein such as a water-soluble resin, wherein the water-soluble resin dissolves in an aqueous solution having a temperature equal to or above 40 degrees Celsius, wherein the disposal bag is disintegrated and/or dissolved at a temperature equal to or above 65 degrees Celsius.

Figure 7B shows a side view of the disposal bag 101 when the disposal bag 101 is in a configuration about to be closed.

It will be appreciated that the disposal bag described herein may be provided with alternative fastening members such as a zip lock or other types of snap-in fasteners fastening members. Further, the disposal bag described herein may have a size suitable for accommodating one or more hygienic absorbent articles such as diapers described herein.

### Determination of water solubility

The water solubility described herein, such as the water solubility of an object such as the fastening member described herein, may be determined as follows. The object with a weight of 10 grams (g) is put into a 500 milliliter (mL) vessel. Water is added in an amount of 30 times the object weight. The water may be stirred during the examination. The temperature of the water or the aqueous solution may be adjusted as desired and the examination may take place immediately or after a desired time such as one hour. The examination may take place by ocular inspection. Additionally or alternatively, the water solubility may be determined as described in the Example section of this document.

The disclosure is illustrated by the following non-limitative Example.

### EXAMPLE

### Purpose:

The purpose of the test was to find at which temperature and time the selected polymers was completely dissolved.

### Test set up:

950 ml water at room temperature was mixed with 50 g polymer resin during stirring.

The water was gradually heated and when the polymer started to dissolve the temperature was stabilized.

The time needed and the temperature when the polymer was dissolved was recorded. The solution was then poured through a 500 micron sieve. The polymer was considered dissolved when the liquid passed through without leaving residues in the sieve.

### Polymers tested:

The following polyvinyl alcohol polymers were tested:
- Mowiflex H15, lot no: P 17-6-0030
- Mowiflex C600, lot no: P 17-5-0029
- Mowiflex M 05, lot no: DS 1116.1

### Supplier Kuraray Europe GmbH.

### Equipment used:

A magnetic stirrer with heated plate, RCT basic, IKA Werke.
A digital thermometer, Testo 922, Testo AG
2000 ml beaker, Schott
Balance, BCE 6202-1S Sartorius Laboratory instruments GmbH & Co. KG
Sieve 500 micron, Retch Analysen Sieb

### Results:

### Dissolution test 1, Mowiflex H15.

The polymer was completely dissolved at 83 degr. C. Time needed, 30 minutes. Dissolution test 2, Mowiflex C600.
The polymer was completely dissolved at 42 degr. C. Time needed, 20 minutes. Dissolution test 3, Mowiflex M05.
The polymer was completely dissolved at 80 degr. C. Time needed, 40 minutes.

## Claims

1. A hygienic absorbent article (1) comprising a fastening member (5) which comprises a water-soluble resin, wherein the water-soluble resin dissolves in an aqueous solution having a temperature equal to or above 40 degrees Celsius, wherein the fastening member is disintegrated and/or dissolved at a temperature equal to or above 40 degrees Celsius while remaining undissolved at a temperature below 40 degrees Celsius, wherein the aqueous solution has a temperature within a range of from 40 degrees Celsius to 100 degrees Celsius, such as from 60 degrees Celsius to 100 degrees Celsius, such as from 80 degrees Celsius to 100 degrees Celsius, and wherein the fastening member is a mechanical fastening member with cooperating fastening elements,
wherein the water-soluble resin comprises polyvinyl alcohol,
the polyvinyl alcohol is derived from polyvinyl acetate, and
the polyvinyl alcohol comprises a degree of hydrolysis from 70 mole% to 100 mole%.

2. The hygienic absorbent article (1) according to claim 1, wherein the aqueous solution comprises water and optionally alcohol such as ethanol.

3. The hygienic absorbent article (1) according to claim 1 or 2, wherein the polyvinyl alcohol comprises a degree of hydrolysis from 85 mole% to 100 mole%, from 70 mole% to 90 mole%, or from 70 mole% to 80 mole% .

4. The hygienic absorbent article (1) according to any one of claims 1-3, wherein the polyvinyl alcohol comprises a degree of hydrolysis from 99 mole% to 100 mole%.

5. The hygienic absorbent article (1) according to any one of claims 1-3, wherein the polyvinyl alcohol comprises a degree of hydrolysis from 98 mole% to 99 mole %.

6. The hygienic absorbent article (1) according to any one of claims 1-3, wherein the polyvinyl alcohol comprises a degree of hydrolysis from 90 mole% to 98 mol%.

7. The hygienic absorbent article (1) according to any one of claims 1-3, wherein the polyvinyl alcohol comprises a degree of hydrolysis from 85 mole % to 90 mol%.

8. The hygienic absorbent article (1) according to any one of claims 1-7, wherein the polyvinyl alcohol has a molecular weight from 13.000 g/mole to 186.000 g/mole.

9. The hygienic absorbent article (1) according to any one of the preceding claims, which does not comprise an anti-foaming agent.

10. The hygienic absorbent article (1) according to any one of the preceding claims, wherein the fastening member is a disposal fastening member.

## Patentansprüche

1. Hygienischer absorbierender Artikel (1), umfassend ein Befestigungselement (5), das ein wasserlösliches Harz umfasst, wobei sich das wasserlösliche Harz in einer wässrigen Lösung mit einer Temperatur gleich oder über 40 Grad Celsius löst, wobei das Befestigungselement bei einer Temperatur gleich oder über 40 Grad Celsius zerfällt und/oder sich löst während es bei einer Temperatur unter 40 Grad Celsius ungelöst bleibt, wobei die wässrige Lösung eine Temperatur innerhalb eines Bereichs von 40 Grad Celsius bis 100 Grad Celsius, wie von 60 Grad Celsius bis 100 Grad Celsius, wie von 80 Grad Celsius bis 100 Grad Celsius, aufweist, und wobei das Befestigungselement ein mechanisches Befestigungselement mit zusammenwirkenden Befestigungselementen ist,
wobei das wasserlösliche Harz Polyvinylalkohol umfasst,
der Polyvinylalkohol von Polyvinylacetat abgeleitet ist, und der Polyvinylalkohol einen Hydrolysegrad von 70 Mol-% bis 100 Mol-% aufweist.

2. Hygienischer absorbierender Artikel (1) gemäß Anspruch 1, wobei die wässrige Lösung Wasser und optional Alkohol wie Ethanol umfasst.

3. Hygienischer absorbierender Artikel (1) gemäß Anspruch 1 oder 2, wobei der Polyvinylalkohol einen Hydrolysegrad von 85 Mol-% bis 100 Mol-%, von 70 Mol-% bis 90 Mol-% oder von 70 Mol-% bis 80 Mol-% aufweist.

4. Hygienischer absorbierender Artikel (1) gemäß mindestens einem der Ansprüche 1-3, wobei der Polyvinylalkohol einen Hydrolysegrad von 99 Mol-% bis 100 Mol-% aufweist.

5. Hygienischer absorbierender Artikel (1) gemäß mindestens einem der Ansprüche 1-3, wobei der Polyvinylalkohol einen Hydrolysegrad von 98 Mol-% bis 99 Mol-% aufweist.

6. Hygienischer absorbierender Artikel (1) gemäß mindestens einem der Ansprüche 1-3, wobei der Polyvinylalkohol einen Hydrolysegrad von 90 Mol-% bis 98 Mol-% aufweist.

7. Hygienischer absorbierender Artikel (1) gemäß mindestens einem der Ansprüche 1-3, wobei der Polyvinylalkohol einen Hydrolysegrad von 85 Mol-% bis 90 Mol-% aufweist.

8. Hygienischer absorbierender Artikel (1) gemäß mindestens einem der Ansprüche 1-7, wobei der Polyvinylalkohol ein Molekulargewicht von 13.000 g/mol bis 186.000 g/mol aufweist.

9. Hygienischer absorbierender Artikel (1) gemäß mindestens einem der vorhergehenden Ansprüche, der kein Antischaummittel umfasst.

10. Hygienischer absorbierender Artikel (1) gemäß mindestens einem der vorhergehenden Ansprüche, wobei das Befestigungselement ein Entsorgungsbefestigungselement ist.

## Revendications

1. Article absorbant hygiénique (1) comprenant un élément de fixation (5) qui comprend une résine soluble dans l'eau, dans lequel la résine soluble dans l'eau se dissout dans une solution aqueuse ayant une température égale ou supérieure à 40 degrés Celsius, dans lequel l'élément de fixation est désintégré et/ou dissous à une température égale ou supérieure à 40 degrés Celsius tout en restant non dissous à une température inférieure à 40 degrés Celsius, dans laquelle la solution aqueuse a une température comprise dans un domaine de 40 à 100 degrés Celsius, par exemple de 60 à 100 degrés Celsius, par exemple de 80 à 100 degrés Celsius, et dans lequel l'élément de fixation est un élément de fixation mécanique avec des éléments de fixation coopératifs, dans lequel la résine soluble dans l'eau comprend de l'alcool polyvinylique,
l'alcool polyvinylique est dérivé de l'acétate de polyvinyle, et
l'alcool polyvinylique comprend un degré d'hydrolyse de 70 mole% à 100 mole%.

2. L'article absorbant hygiénique (1) selon la revendication 1, dans lequel la solution aqueuse comprend de l'eau et optionnellement de l'alcool tel que l'éthanol.

3. L'article absorbant hygiénique (1) selon la revendication 1 ou 2, dans lequel l'alcool polyvinylique comprend un degré d'hydrolyse de 85 mole% à 100 mole%, de 70 mole% à 90 mole%, ou de 70 mole% à 80 mole%.

4. L'article absorbant hygiénique (1) selon l'une quelconque des revendications 1 à 3, dans lequel l'alcool polyvinylique comprend un degré d'hydrolyse de 99 mole% à 100 mole%.

5. L'article absorbant hygiénique (1) selon l'une quelconque des revendications 1 à 3, dans lequel l'alcool polyvinylique comprend un degré d'hydrolyse de 98 mole % à 99 mole %.

6. L'article absorbant hygiénique (1) selon l'une quelconque des revendications 1 à 3, dans lequel l'alcool polyvinylique comprend un degré d'hydrolyse de 90 mole % à 98 mole %.

7. L'article absorbant hygiénique (1) selon l'une quelconque des revendications 1 à 3, dans lequel l'alcool polyvinylique comprend un degré d'hydrolyse de 85 mole % à 90 mole %.

8. L'article absorbant hygiénique (1) selon l'une des revendications 1 à 7, dans lequel l'alcool polyvinylique a un poids moléculaire de 13000 g/mole à 186000 g/mole.

9. L'article absorbant hygiénique (1) selon l'une quelconque des revendications précédentes, qui ne comprend pas d'agent anti-mousse.

10. L'article absorbant hygiénique (1) selon l'une des revendications précédentes, dans lequel l'élément de fixation est un élément de fixation pour élimination.
